# FASCICULE DE BREVET EUROPEEN

(11) **EP 4 208 112 B1**
(45) Date de publication et mention de la délivrance du brevet: **28.01.2026**
(21) Numéro de dépôt: 21777568.3
(22) Date de dépôt: 24.08.2021
(51) Int. Cl.: A61B 17/80, A61B 34/10

(54) **PROCÉDÉ DE GÉNÉRATION DE MODÈLES NUMÉRIQUES DE PLAQUES D'OSTÉOSYNTHÈSE SPÉCIFIQUES À LA MORPHOLOGIE DU PATIENT**
VERFAHREN ZUR ERZEUGUNG VON DIGITALEN MODELLEN FÜR DIE MORPHOLOGIE DES PATIENTENSPEZIFISCHEN OSTEOSYNTHESEPLATTEN
METHOD FOR GENERATING DIGITAL MODELS OF OSTEOSYNTHESIS PLATES SPECIFIC TO THE MORPHOLOGY OF THE PATIENT

(30) Priorité: 02.09.2020 FR 2008889
(43) Date de publication de la demande: 12.07.2023
(73) Titulaire: Abys Medical, 17000 La Rochelle (FR)
(72) Inventeur: DESTAINVILLE, Arnaud, 17138 SAINT-XANDRE (FR); RICHART, Olivier, LE BOIS-PLAGE- 17580 (FR)
(74) Mandataire: Semaoune, Idriss
(86) Numéro de dépôt international: PCT/FR2021/051480
(87) Numéro de publication internationale: WO 2022/049333

(56) Documents cités:
- EP-A1- 3 080 619
- EP-A1- 3 384 865
- WO-A1-2020/139809

## Description

### Domaine technique

La présente divulgation est relative à un procédé de fabrication de plaque d'ostéosynthèse, ainsi qu'à des plaques d'ostéosynthèse ainsi fabriquées.

### Etat de la technique

Les utilisateurs ou les patients recevant des soins d'un chirurgien ou d'un opérateur sont souvent traités avec une vision uniforme, en particulier en ce qui concerne les opérations chirurgicales courantes. Cette vision s'étend aux implants ou aux dispositifs utilisés pour chaque patient, même si chaque patient est différent. Cela s'explique en partie par le fait qu'il n'est pas pratique de produire des matériaux personnalisés pour chaque patient en raison des coûts, du temps et des limitations technologiques.

La description du contexte fournie ici a pour but de présenter de manière générale le contexte de la divulgation. Les travaux des inventeurs actuellement nommés, dans la mesure où ils sont décrits dans cette section, ainsi que les aspects de la description qui ne peuvent pas être considérés comme faisant partie de l'état de la technique au moment du dépôt, ne sont pas expressément ou implicitement admis comme faisant partie de l'état de la technique dans le cadre de la présente divulgation.

EP3 080 619 A1 divulgue un procédé de navigation chirurgicale ainsi qu'une méthode de conception d'une plaque traumatique, c'est-à-dire d'un implant destiné à être fixé sur un os. Ce procédé repose sur la création d'un modèle 3D de l'anatomie du patient à partir d'images obtenues par rayons X. Ce modèle est ensuite comparé à un modèle générique afin d'identifier d'éventuels défauts anatomiques et de proposer une reconstruction personnalisée adaptée à la morphologie du patient.

### Exposé de l'invention

Selon un premier aspect de l'invention, il est proposé, un procédé de génération d'une plaque d'ostéosynthèse convenant à être fixée sur un os comportant les étapes suivantes : obtenir un modèle d'os représentant un os généré à partir de données d'imagerie correspondant à l'os ; déterminer un ensemble de points d'entrée virtuels sur le modèle osseux ; générer une surface virtuelle adjacente au modèle osseux sur la base du modèle osseux et de l'ensemble des points d'entrée virtuels ; générer un modèle de plaque d'ostéosynthèse basé sur la surface virtuelle ; créer un trou virtuel à chaque point d'entrée virtuel de l'ensemble des points d'entrée virtuels le long du modèle de plaque d'ostéosynthèse ; et transmettre le modèle de plaque d'ostéosynthèse à une machine de fabrication de plaques d'ostéosynthèse.

Dans la présente description, un modèle d'os vise une représentation réaliste 2D ou 3D de l'os, c'est-à-dire qui ne comporte pas d'approximation de type modélisation mathématique.

Avantageusement, l'ensemble des points d'entrée virtuels peuvent indiquer les endroits où une vis est insérée dans l'os.

De préférence, le modèle osseux est tridimensionnel.

Le procédé peut comporter en outre une étape d'obtention de données d'imagerie correspondant à l'os à partir d'un dispositif d'imagerie.

La surface virtuelle peut comprendre l'ensemble des emplacements d'entrée virtuels, une vis est l'une des multiples vis et le trou virtuel est l'un des multiples trous.

Le modèle de plaque d'ostéosynthèse peut comprendre une face virtuelle définie par la surface virtuelle et une épaisseur définie par une extrusion de la face virtuelle.

Le procédé peut en outre comporter la génération d'une scène virtuelle comprenant le modèle d'os, dans lequel le modèle de plaque d'ostéosynthèse est pressé contre le modèle d'os et positionné de telle sorte qu'une face virtuelle du modèle de plaque d'ostéosynthèse coïncide avec la surface virtuelle ; et dans lequel la génération d'un modèle de plaque d'ostéosynthèse et la fabrication ultérieure d'une plaque d'ostéosynthèse sur mesure évitent au chirurgien de devoir modifier la plaque d'ostéosynthèse pendant l'opération.

Avantageusement, la surface virtuelle peut être déterminée électroniquement à partir d'une courbe génératrice virtuelle qui passe successivement par des points de référence, les points de référence étant déterminés comme les centres de l'ensemble des points d'entrée virtuels et d'au moins deux courbes virtuelles latérales qui sont disposées de chaque côté de la courbe génératrice virtuelle.

Selon une possibilité, les points de référence comprennent des extrémités longitudinales, la courbe génératrice virtuelle passant successivement par l'une des extrémités longitudinales, les centres de l'ensemble des points d'entrée virtuels, et une autre des extrémités longitudinales.

La courbe génératrice virtuelle peut être une spline de type géodésique passant par les points de référence.

Les courbes virtuelles latérales peuvent être des splines de type géodésique qui passent par des images des points de référence, et les images peuvent être obtenues par translation d'une distance qui est fonction d'une largeur d'une vis à un diamètre maximal perpendiculaire à un axe du modèle de plaque d'ostéosynthèse et tangente à la surface virtuelle du modèle d'os.

Les courbes virtuelles latérales peuvent être des courbes virtuelles espacées d'une distance prédéterminée de part et d'autre de la courbe virtuelle génératrice et projetées sur la surface virtuelle de l'os.

Le procédé peut comporter en outre une détermination d'un modèle de plaque d'ostéosynthèse intermédiaire en ajoutant des congés virtuels au modèle de plaque d'ostéosynthèse aux extrémités longitudinales du modèle de plaque d'ostéosynthèse, dans lequel la détermination du modèle de plaque d'ostéosynthèse est effectuée en modifiant le modèle de plaque d'ostéosynthèse intermédiaire.

La machine de fabrication de plaques d'ostéosynthèse peut être une machine de fabrication additive ou soustractive.

La plaque d'ostéosynthèse qui est fabriquée peut-être faite de matériaux de grade médical.

La machine de fabrication de plaques d'ostéosynthèse peut utiliser le frittage laser ou l'impression tridimensionnelle et se trouve à un endroit éloigné de la modélisation.

Selon un deuxième aspect de l'invention, il est proposé un produit programme d'ordinateur comprenant des instructions qui, lorsque le programme est exécuté par un ordinateur, conduisent celui-ci à mettre en œuvre les étapes du procédé selon le premier aspect de l'invention, ou l'un ou plusieurs de ses perfectionnements.

Selon un troisième aspect de l'invention, il est proposé un support d'enregistrement lisible par ordinateur comprenant des instructions qui, lorsqu'elles sont exécutées par un ordinateur, conduisent celui-ci à mettre en œuvre les étapes du procédé selon le premier aspect de l'invention, ou l'un ou plusieurs de ses perfectionnements.

Selon un quatrième aspect de l'invention, il est proposé un procédé de génération d'un modèle chirurgical, comprenant les étapes suivantes : obtention de données d'imagerie ; génération d'un modèle osseux basé sur les données d'imagerie d'un os ; construction d'une surface virtuelle sur un visage le modèle d'os en utilisant le modèle d'os ; réception d'une position de forage sur le modèle osseux ; génération d'un modèle chirurgical basé sur la surface virtuelle et la position du foret ; et envoi du modèle chirurgical à une machine de fabrication de modèles chirurgicaux.

Le modèle chirurgical peut par exemple être un modèle de plaque d'ostéosynthèse, un guide chirurgical, ou à la fois un modèle de plaque d'ostéosynthèse et un guide chirurgical.

La machine de fabrication de modèles chirurgicaux peut être une machine de fabrication additive ou soustractive.

Le modèle chirurgical qui est fabriqué peut-être constitué de matériaux de grade médical.

Selon un quatrième aspect de l'invention, il est proposé un système de génération d'un modèle chirurgical, comprenant : une interface utilisateur graphique ; au moins un processeur ; et une mémoire couplée à au moins un processeur, dans laquelle la mémoire stocke des instructions exécutées par le ou les processeurs.

Les instructions comprennent la mise en œuvre des étapes suivantes : une récupération d'un modèle d'os à partir des données d'imagerie correspondant à un os ; un affichage, via un écran, du modèle osseux ; une réception de données, via l'affichage, indiquant un emplacement d'entrée virtuel sur le modèle osseux ; une construction d'une surface virtuelle adjacente au modèle osseux sur la base du modèle osseux et de l'emplacement d'entrée virtuel ; une création d'un modèle de plaque d'ostéosynthèse basé sur la surface virtuelle ; un forage d'un trou virtuel à l'emplacement d'entrée virtuel sur le modèle de plaque d'ostéosynthèse ; et une transmission du modèle de plaque d'ostéosynthèse à un stockage de modèle de plaque.

Le modèle de plaque d'ostéosynthèse peut comprendre une face virtuelle définie par la surface virtuelle et une épaisseur définie par une extrusion de la face virtuelle.

Les instructions peuvent comprendre la mise en œuvre des étapes suivantes : une génération d'une scène virtuelle comprenant le modèle d'os, dans laquelle le modèle de plaque d'ostéosynthèse est pressé contre le modèle d'os et positionné de telle sorte qu'une face virtuelle du modèle de plaque d'ostéosynthèse coïncide avec la surface virtuelle, et dans lequel la génération d'un modèle de plaque d'ostéosynthèse et la fabrication ultérieure d'une plaque d'ostéosynthèse sur mesure évite au chirurgien de devoir modifier la plaque pendant l'opération.

La surface virtuelle peut être déterminée électroniquement à partir d'une courbe génératrice virtuelle qui passe successivement par des points de référence, les points de référence étant déterminés comme les centres de la position de forage et d'au moins deux courbes virtuelles latérales qui sont disposées de part et d'autre de la courbe génératrice virtuelle.

Les points de référence peuvent comprendre des extrémités longitudinales, la courbe génératrice virtuelle passant successivement par l'une des extrémités longitudinales, les centres de la position de forage et une autre des extrémités longitudinales.

### Brève description des figures

D'autres caractéristiques et avantages de la présente divulgation apparaîtront au cours de la lecture de la description détaillée qui va suivre pour la compréhension de laquelle on se reportera aux dessins annexés dans lesquels :
La figure 1 est une vue en perspective d'une représentation d'un modèle tridimensionnel d'os dans lequel des vis virtuelles sont positionnées,
La figure 2 est une vue semblable à Figure 1, les vis virtuelles étant enlevées de l'os, ne laissant subsister qu'une trace d'entrées virtuelles des vis virtuelle sur la surface de l'os.
La figure 3 est une vue semblable à Figure 2, comportant en outre des extrémités longitudinales.
La figure 4 est une vue semblable à Figure 3, comportant en outre une courbe génératrice passant par des points de référence.
La figure 5 est une vue semblable à Figure 4, comportant en outre deux courbes latérales encadrant la courbe génératrice.
La figure 6 est une vue semblable à Figure 5, comportant une surface virtuelle déterminée par les deux courbes latérales de Figure 4.
La figure 7 comporte trois sous-figures semblables à Figure 1, comportant un modèle tridimensionnel d'os et un modèle de plaque d'ostéosynthèse.
La figure 8 comporte deux sous-figures semblables à Figure 1, comportant un modèle tridimensionnel d'os et un modèle de plaque d'ostéosynthèse percée.
La figure 9 comporte un modèle tridimensionnel de plaque percée.
La figure 10 est un diagramme bloc de haut niveau d'un système de génération de plaque selon la présente divulgation.
La figure 11 est un exemple de machine de fabrication additive créant une plaque d'ostéosynthèse perforée.
La figure 12 est un organigramme décrivant la génération d'un modèle de plaque d'ostéosynthèse perforée.

Ces modes de réalisation n'étant nullement limitatifs, on pourra notamment considérer des variantes de la présente divulgation ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier la présente divulgation par rapport à l'état de la technique antérieure. Cette sélection comprend au moins une caractéristique de préférence fonctionnelle sans détail structurel, et/ou avec seulement une partie des détails structurels si cette partie uniquement est suffisante pour conférer un avantage technique ou à différencier la présente divulgation par rapport à l'état de la technique antérieure.

Dans la suite de la description, des éléments présentant une structure identique ou des fonctions analogues seront désignés par de mêmes références.

### Problèmes techniques adressés par la présente divulgation

Une plaque d'ostéosynthèse, aussi parfois désignée par le terme plaque osseuse dans la littérature, est généralement utilisée pour maintenir différentes parties d'un os fracturé ou autrement sectionné sensiblement stationnaires les unes par rapport aux autres pendant et/ou après le processus de consolidation au cours duquel l'os se répare. Les fractures d'un os dans la région de la tête peuvent être particulièrement gênantes en raison de mouvements et/ou de la présence de tissus mous dans la région ostéoarticulaire.

Typiquement, une plaque d'ostéosynthèse orthopédique peut comporter une partie allongée pouvant être fixée à un corps de l'os à l'aide d'une pluralité de vis à ancrage osseux, ladite partie allongée définissant un axe longitudinal, une partie évasée pouvant être fixée à une tête de l'os à l'aide d'au moins une des vis à os, et une partie intermédiaire interconnectant ladite partie allongée et ladite partie évasée.

Plusieurs dimensions de plaques peuvent être prévues, présentant des largeurs et des longueurs différentes. Néanmoins, elles ne sont pas exactement adaptées à chaque anatomie. Souvent le chirurgien utilise une plaque droite et la déforme. La plaque peut avoir une forme à peu près anatomique, de base avec une courbure, mais cette courbure n'est pas adaptée à chaque morphologie.

Il existe en effet une importante variabilité anatomique du squelette inter- et intrapopulationnelle nécessitant de définir des intervalles dimensionnels larges ainsi que des conceptions dédiées pour les systèmes de contention afin de répondre à l'ensemble des situations rencontrées par les praticiens. Il est également proposé que le chirurgien pratique lui-même la déformation anatomique des plaques proposées afin d'optimiser le contact à l'os. Cette pratique n'est néanmoins guère satisfaisante, car souvent imprécise et chronophage, une situation à risque pendant une intervention chirurgicale. Cette pratique nécessite par ailleurs que le praticien déforme à l'aide de pinces massives du matériel métallique qui peut se trouver endommagé lorsque soumis à de telles contraintes, ce qui peut laisser augurer d'un risque sur les suites opératoires.

La variabilité des conceptions doit par ailleurs intégrer le nombre élevé de typologies de fractures squelettiques recensées (os longs, os rachidiens, fracture simple ou complexe, extra articulaire ou intraarticulaire, transverse, multi-fragmentaire...) en plus des ostéotomies qui, par nature, sont provoquées. Rien que pour les fractures d'os longs, la classification réalisée par Müller recense plus de 120 fractures différentes dont au moins 50 sont classiquement traitées par ostéosynthèse.

La conséquence de l'ensemble de ces exigences, augmentées des possibilités variées de matériaux de fabrication, est aujourd'hui la mise à disposition d'une quantité pléthorique de dispositifs d'ostéosynthèse aux chirurgiens orthopédistes et traumatologues humains ou vétérinaires. Il n'est ainsi pas rare qu'un praticien doive choisir pendant l'intervention entre plusieurs dizaines de plaques et de vis pour réaliser son intervention, associées à plusieurs instruments spécialement conçus pour la pose des implants associés. En plus du temps que doit prendre le praticien pour affiner son analyse préopératoire afin de déterminer les références dont il a besoin parmi les dizaines proposées, il faut intégrer, en amont et en aval, la pesanteur de gestion de ce stock, qu'il s'agisse de l'impact financier et administratif ou de la gestion du nettoyage et de la stérilisation de ces dispositifs. Du fait du choix historique de l'offre pléthorique pour répondre à l'ensemble des besoins des praticiens orthopédistes et traumatologues, l'impact opérationnel pour les établissements de santé est indissociable de l'analyse.

La fabrication de systèmes d'ostéosynthèse sur mesure, et plus particulièrement des plaques, est donc une solution pour mieux répondre aux attentes des praticiens sur le plan chirurgical, mais doit également permettre, par extension et par un processus de fabrication adapté et compétitif et des instruments chirurgicaux simples et attractifs, d'être intégré dans une offre plus large qui devra également optimiser l'impact opérationnel au sein des établissements de santé et des équipes médicales.

La présente divulgation apporte une solution à ces problèmes en proposant un procédé de génération de fichiers numériques de plaques spécifiques à l'anatomie du patient en vue de leur fabrication.

Il est aujourd'hui possible de planifier une intervention à partir de représentations 3D de l'os à traiter. Ces représentations 3D peuvent, par exemple, être obtenues à partir de données scanographiques obtenues par tomodensitométrie ou encore d'imagerie par résonnance magnétique (IRM). Cette planification est généralement accompagnée d'une simulation des différentes étapes de l'intervention, en particulier, la position et les angles de différents plans de coupe (cas de l'ostéotomie de correction des os déformés) ou le repositionnement de fragments osseux les uns par rapport aux autres (cas des fractures).

Afin d'aider le chirurgien à respecter au plus près le plan chirurgical préétabli dans le cas d'une ostéotomie de correction par exemple, des guides chirurgicaux dédiés et personnalisés sont parfois mis à la disposition des praticiens. Ces guides de coupe présentent des surfaces de fixation aux os épousant la forme de ceux-ci, ce qui permet de positionner les plans de coupe et les perçages de façon précise et univoque.

Néanmoins, l'état de la technique ne permet pas de proposer des plaques de contention osseuse (d'ostéosynthèse) conçues sur mesure préalablement à leur fabrication.

### Introduction

Il est proposé, selon un premier aspect de la présente divulgation, un procédé de génération d'une plaque d'ostéosynthèse convenant à être fixée sur un os comportant : une étape d'obtention d'un modèle d'os tridimensionnel représentant l'os, à partir d'un dispositif d'imagerie permettant d'imager ledit os, des données tridimensionnelles de l'os et, sur la base des données tridimensionnelles obtenues, une étape de réception d'emplacements virtuels d'entrée d'une pluralité d'objets d'ancrages virtuels, à partir d'une représentation tridimensionnelle comportant une représentation d'un os virtuel générée à partir du modèle d'os tridimensionnel à laquelle est ajoutée une pluralité d'objets d'ancrages virtuels, et une étape de génération d'une surface virtuelle disposée sur l'os virtuel, la surface virtuelle étant générée à partir des emplacements virtuels d'entrée de la pluralité d'objets d'ancrages virtuels, ladite surface virtuelle comportant les emplacements virtuels d'entrée.

Le procédé comporte une étape de génération d'un modèle de plaque d'ostéosynthèse, la plaque d'ostéosynthèse présentant une face virtuelle définie par la surface virtuelle et une épaisseur obtenue par extrusion de la face virtuelle, une étape de génération d'une scène virtuelle comportant l'os virtuel, le modèle de plaque d'ostéosynthèse virtuelle plaqué sur l'os virtuel et positionné de sorte que sa face virtuelle coïncide avec la surface virtuelle, une étape de détermination d'un deuxième modèle de plaque d'ostéosynthèse par modification du modèle de plaque d'ostéosynthèse par perçage virtuel de la plaque d'ostéosynthèse sur son épaisseur pour former des trous d'objets d'ancrages virtuels agencés pour recevoir la pluralité d'objets d'ancrages virtuels, une étape d'envoi du modèle de plaque d'ostéosynthèse modifiée à destination d'un dispositif permettant de fabriquer une plaque d'ostéosynthèse à partir du modèle de plaque d'ostéosynthèse modifiée.

Le dispositif d'imagerie peut par exemple être un scanner tomodensitométrique ou une IRM. Les données tridimensionnelles de l'os peuvent par exemple être reconstruites à partir de fichiers au format DICOM, pour l'anglais *Digital imaging and communications in medicine,* qui est une norme standard pour la gestion informatique des données issues de l'imagerie médicale. Les objets d'ancrages virtuels peuvent par exemple être des vis virtuelles. Alternativement, ou en complément, des objets d'ancrages peuvent être des broches, ou tout autre type d'implants pouvant être rapportés dans la plaque.

Les objets d'ancrages virtuels, tels que des vis virtuelles, sont des éléments volumiques à base polygones. Typiquement, de tels objets d'ancrages virtuels sont formés d'une pluralité de mesh. Un mesh ou maillage est un objet tridimensionnel constitué de sommets, d'arêtes et de faces organisés en polygones sous forme de fil de fer dans une infographie tridimensionnelle. Les faces se composent généralement de triangles, de quadrilatères ou d'autres polygones convexes simples, car cela simplifie le rendu. Les faces peuvent être combinées pour former des polygones concaves plus complexes, ou des polygones avec des trous. Les objets d'ancrages virtuels peuvent être de différentes dimensions, en longueur ou en diamètre. Les vis d'ancrages virtuels peuvent présenter différents types d'empreintes, et éventuellement plusieurs étages d'empreintes présentant éventuellement plusieurs types d'empreintes. Les vis virtuelles peuvent être de type cortical, spongieux, verrouillé ou non.

La détermination des emplacements virtuels d'entrée des objets d'ancrages virtuels peut par exemple être réalisée par un utilisateur. À cet effet, l'utilisateur peut préciser lesdits emplacements d'entrée sur une représentation du modèle d'os. Selon une première possibilité, la détermination d'emplacements virtuels de sortie des objets d'ancrages virtuels peut être réalisée par l'utilisateur. À cet effet, l'utilisateur peut préciser lesdits emplacements de sortie sur une représentation du modèle d'os.

Selon une deuxième possibilité, l'orientation des objets d'ancrages virtuels peut être déterminée de sorte qu'une fois positionné à l'emplacement virtuel d'entrée selon ladite orientation, l'objet d'ancrage virtuel est dirigé vers le centre du canal médullaire de l'os. Dit autrement, l'objet d'ancrage virtuel est dirigé vers le barycentre du canal médullaire de l'os, à la perpendiculaire de l'axe longitudinal passant par l'emplacement virtuel d'entrée.

Selon une variante, les objets d'ancrages virtuels peuvent être positionnés à partir de traitement effectué par une unité de calcul à partir du modèle d'os tridimensionnel. En complément, le positionnement des objets d'ancrages virtuels positionnés par l'unité de calcul peut être validé, ou modifié, par un chirurgien qui possède seul la qualification à la réalisation d'un geste pouvant affecter le choix thérapeutique. De préférence, les emplacements virtuels d'entrée de la pluralité d'objets d'ancrages virtuels sont disposés dans l'intérieur topologique de la surface virtuelle. Par extrusion de la face virtuelle, la présente description vise le procédé d'obtention d'un objet tridimensionnel à partir d'une surface tridimensionnelle. Il s'agit d'une terminologie courante, par exemple dans le domaine des images vectorielles.

Selon une possibilité, la surface virtuelle est déterminée à partir d'une courbe virtuelle génératrice passant successivement par des points de références, les points de référence étant déterminés comme les centres des emplacements virtuels d'entrée et d'au moins deux courbes virtuelles latérales disposées sur l'os de part et d'autre de la courbe virtuelle.

Le procédé selon la présente divulgation peut en outre comporter un positionnement sur la surface du modèle d'os tridimensionnel virtuel d'extrémités longitudinales d'un modèle de plaque virtuelle, lesdites extrémités longitudinales étant déterminées à partir des emplacements d'entrée de la pluralité d'objets d'ancrages virtuels et positionnées sur la surface du modèle tridimensionnel d'os.

Lorsque des extrémités longitudinales sont positionnées sur la surface du modèle d'os tridimensionnel, le procédé selon la présente divulgation peut en outre comprendre une étape de détermination d'un modèle de plaque d'ostéosynthèse intermédiaire par ajout de congés virtuels sur le modèle de plaque d'ostéosynthèse au niveau des extrémités longitudinales, la détermination du deuxième modèle de plaque d'ostéosynthèse étant réalisée par modification dudit modèle de plaque d'ostéosynthèse intermédiaire.

Lorsque des extrémités longitudinales sont positionnées sur la surface du modèle d'os tridimensionnel, les points de référence comportent en outre les extrémités longitudinales, la courbe virtuelle génératrice passant successivement par l'une des extrémités longitudinales, les centres des emplacements virtuels d'entrée, et une autre des extrémités longitudinales. La courbe virtuelle peut être une spline qui est une géodésique passant par les points de références sur la surface de l'os virtuel.

Selon une première possibilité, les courbes virtuelles latérales peuvent être des splines passant par des images des points de référence, lesdites images étant obtenues par une translation d'une distance dépendante de la largeur de la vis de plus grand diamètre, perpendiculairement à l'axe de la plaque et tangent à la surface de l'os. Aussi, les point-images sont tangents à la surface de l'os et sont décalés d'une demi-largeur de plaque perpendiculairement à la spline. Selon une autre possibilité, éventuellement combinable avec la première, les courbes virtuelles latérales sont des courbes virtuelles espacées d'une distance prédéterminée de part et d'autre de la courbe virtuelle génératrice et projetées sur la surface virtuelle de l'os. Lors de l'étape de détermination du deuxième modèle de plaque d'ostéosynthèse, l'étape de perçage virtuel peut être réalisée par soustraction de modèles de trous, qui dépendent avantageusement du type et de la taille de l'objet d'ancrage virtuel, de la plaque d'ostéosynthèse sur son épaisseur pour former des trous d'objets d'ancrages virtuels agencés pour recevoir la pluralité d'objets d'ancrages virtuels.

Le dispositif permettant de fabriquer une plaque d'ostéosynthèse à partir du modèle de plaque d'ostéosynthèse modifié peut être du type machine de fabrication additive, souvent désignée sous le terme générique « d'imprimante 3D ». Il est néanmoins possible de faire appel à des procédés de fabrication traditionnels, tel que l'usinage multiaxe ou le moulage par injection et de reprise pour la réalisation des alésages dans la plaque ainsi fabriquée. La plaque d'ostéosynthèse fabriquée peut être en matériau métallique de grade médical. Ce type de matériau vise notamment l'acier inoxydable 316L, le titane pur ou encore l'alliage de Titane TA6V ou TA6V-Eli.

Selon un deuxième aspect de la présente divulgation, il est proposé un dispositif de génération d'une plaque d'ostéosynthèse convenant à être fixée sur un os comportant une unité de calcul configurée pour : obtenir un modèle d'os tridimensionnel représentant l'os sur la base de données tridimensionnelles obtenues à partir d'un dispositif d'imagerie permettant d'imager ledit os, obtenir des emplacements virtuels d'entrée d'une pluralité d'objets d'ancrages virtuels, à partir d'une représentation comportant une représentation tridimensionnelle d'un os virtuel générée à partir du modèle d'os tridimensionnel à laquelle est ajoutée une pluralité d'objets d'ancrages virtuels positionnés, chacun des objets d'ancrages virtuels étant positionné entre un emplacement virtuel d'entrée sur l'os virtuel, et un emplacement virtuel de sortie sur l'os virtuel, générer une surface virtuelle disposée sur l'os virtuel, la surface virtuelle étant générée à partir des emplacements virtuels d'entrée de la pluralité d'objets d'ancrages virtuels, ladite surface virtuelle comportant les emplacements virtuels d'entrée.

L'unité de calcul peut être configurée pour générer un modèle de plaque d'ostéosynthèse, le modèle plaque d'ostéosynthèse présentant une face virtuelle définie par la surface virtuelle et une épaisseur obtenue par extrusion de la face virtuelle, générer une scène virtuelle comportant l'os virtuel, le modèle de plaque d'ostéosynthèse virtuel plaqué sur l'os virtuel et positionné de sorte que sa face virtuelle coïncide avec la surface virtuelle, déterminer un modèle de plaque d'ostéosynthèse percée par modification du modèle de plaque d'ostéosynthèse par perçage virtuel de la plaque d'ostéosynthèse sur son épaisseur pour former des trous d'objets d'ancrages virtuels agencés pour recevoir la pluralité d'objets d'ancrages virtuels, envoyer le modèle de plaque d'ostéosynthèse percée à destination d'un dispositif permettant de fabriquer une plaque d'ostéosynthèse à partir du modèle de plaque d'ostéosynthèse percée.

Selon un deuxième aspect de la présente divulgation, il est proposé un système de génération d'une plaque d'ostéosynthèse convenant à être fixée sur un os comportant un dispositif de génération de plaque d'ostéosynthèse selon le premier aspect de la présente divulgation, ou l'un ou plusieurs de ses perfectionnements et un dispositif permettant de fabriquer une plaque d'ostéosynthèse à partir du modèle de plaque d'ostéosynthèse percée reçu dudit dispositif de génération.

### Modèle d'os

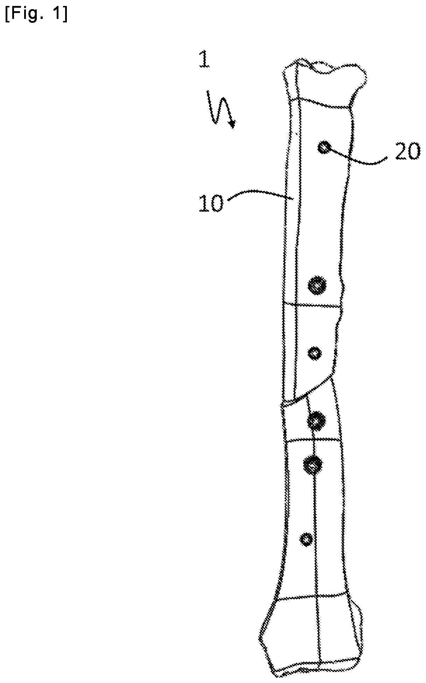

En référence à **Figure** 1, il est représenté une vue en perspective d'une représentation d'un modèle d'os avec vis positionnées 1. Dans la description qui suit, un seul type d'ancrage virtuel est donné, celui de la vis virtuelle. Le modèle d'os avec vis positionnées comporte d'une part un modèle d'os 10, et d'autre part une vis 20. La vis 20 est positionnée dans l'os.

Le modèle tridimensionnel d'os 10 est obtenu à partir de données tridimensionnelles relatives à un os réel, lesquelles sont obtenues à partir d'un dispositif d'imagerie. Les données tridimensionnelles peuvent par exemple être issues d'un format DICOM. Les données tridimensionnelles peuvent être adressées à destination de l'unité de calcul, qui est configurée pour déterminer le modèle tridimensionnel d'os 10 à partir desdites données tridimensionnelles.

Pour obtenir le modèle d'os avec vis positionnée 1, il peut être présenté une représentation du modèle d'os 10 à un utilisateur, par exemple sur un écran d'affichage, tel qu'un écran d'ordinateur ou encore via un dispositif de réalité virtuelle ou de génération d'hologrammes (réalité augmentée ou mixte). L'utilisateur peut sélectionner une vis, ou plusieurs, et la positionner sur la représentation, et donc dans le modèle d'os, générant ainsi le modèle tridimensionnel d'os avec vis positionnées 1.

Pour positionner la vis, il peut être proposé à l'utilisateur d'indiquer un emplacement d'entrée d'une vis sur la représentation du modèle de l'os. L'utilisateur peut par exemple indiquer l'emplacement d'entrée au moyen d'un dispositif de pointage, tel qu'une souris, et en pointant l'emplacement d'entrée de la vis sur la représentation du modèle d'os. Les emplacements d'entrée peuvent être adressés à l'unité de calcul. Selon une possibilité, une orientation de la vis est alors déterminée, de sorte qu'une fois positionnée à l'emplacement d'entrée selon ladite orientation, la vis virtuelle est dirigée vers le centre du canal médullaire de l'os.

Selon une autre possibilité, l'orientation de la vis virtuelle est déterminée par l'utilisateur. Il peut être proposé à l'utilisateur d'indiquer un emplacement de sortie de la vis sur la représentation du modèle de l'os. L'utilisateur peut par exemple indiquer l'emplacement de sortie au moyen d'un dispositif de pointage, tel qu'une souris, et en pointant l'emplacement de sortie de la vis sur la représentation du modèle d'os. Selon encore une autre possibilité, l'orientation de la vis virtuelle est déterminée par l'utilisateur. Il peut être proposé à l'utilisateur d'indiquer un emplacement de sortie de la vis sur la représentation du modèle de l'os. L'utilisateur peut par exemple indiquer l'emplacement de sortie au moyen d'un dispositif de pointage, tel qu'une souris, et en déplaçant sur le modèle l'emplacement de sortie, par exemple en déplaçant la pointe de la vis sur la représentation du modèle d'os.

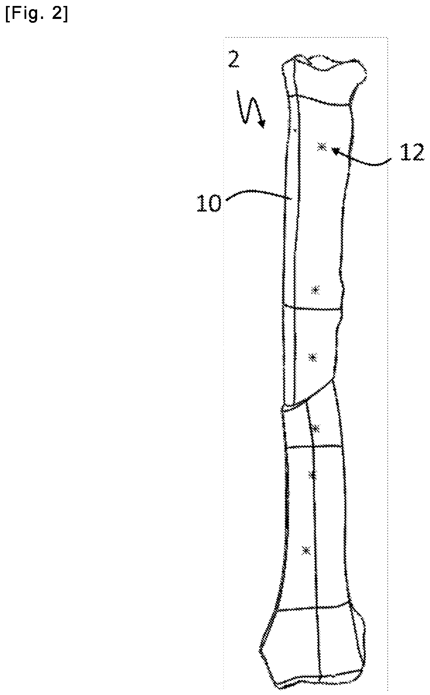

**Figure** 2 représente une vue en perspective d'une représentation d'un modèle d'os avec points d'emplacements d'entrées positionnés 2. Le modèle d'os emplacement d'entrée comporte d'une part le modèle d'os 10, et d'autre un ou plusieurs emplacements d'entrées 12. Plusieurs emplacements d'entrée sont représentés sur la Figure 2. Aussi, l'unité centrale peut recevoir : un modèle tridimensionnel 10 et, des emplacements virtuels d'entrée d'une pluralité de vis virtuelles.

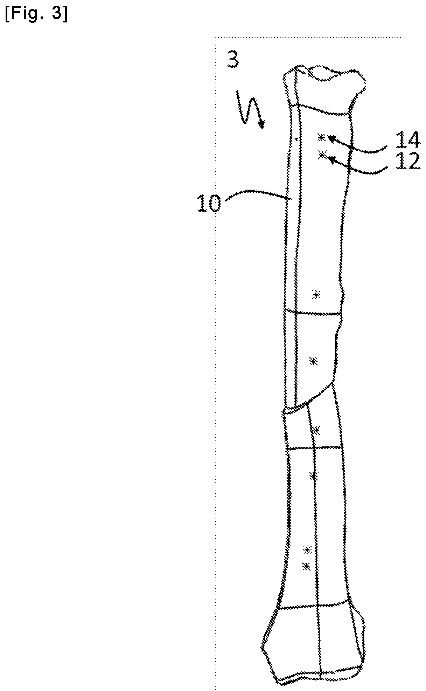

**Figure** 3 représente une vue en perspective d'une représentation d'un modèle d'os avec emplacements d'entrées et extrémités positionnées 3 Le modèle d'os avec emplacement d'entrée comporte d'une part le modèle d'os 10, un emplacement d'entrée 12 et un emplacement d'extrémité longitudinale 14. Deux emplacements d'extrémités longitudinales sont représentés sur la Figure 3. Dans une possibilité de la présente divulgation, les emplacements d'extrémités longitudinales peuvent être déterminés par l'utilisateur.

Alternativement, les emplacements d'extrémités longitudinales peuvent être déterminés par l'unité de calcul, à partir des emplacements d'entrée de la pluralité de vis virtuelles et positionnées sur la surface du modèle d'os. L'emplacement de l'extrémité longitudinale distale est positionné sur la surface de l'os, dans le prolongement des 2 emplacements d'entrées des vis virtuelles les plus distales, d'une distance dépendant de la taille de la vis la plus grosse utilisée. Il en est de même pour l'emplacement de l'extrémité longitudinale proximale.

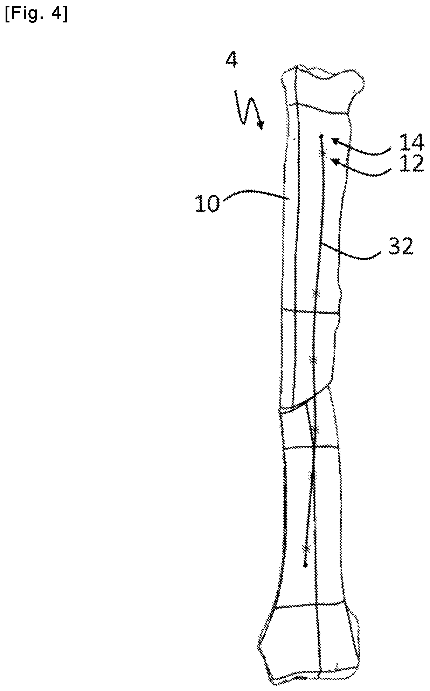

**Figure 4** représente une vue en perspective d'une représentation d'un modèle d'os avec courbe virtuelle génératrice 4. Le modèle d'os avec courbe virtuelle génératrice 4 comporte d'une part le modèle d'os 10, l'emplacement d'entrée 12, l'emplacement d'extrémité longitudinale 14 et une courbe virtuelle génératrice 32. La courbe virtuelle génératrice 32 passe successivement par des points de références. Les points de référence comportent les centres des emplacements virtuels d'entrée 12. Dans l'exemple représenté, les points de référence comportent en outre les extrémités longitudinales 14. La courbe virtuelle génératrice passe successivement par l'une des extrémités longitudinales 14, les centres des emplacements virtuels d'entrée 12, et un autre des extrémités longitudinales 14. La courbe génératrice peut être déterminée comme une spline. En ce cas, les points de référence sont appelés points intermédiaires.

En ce cas, le degré de la spline est typiquement 3. Bien entendu, d'autres degrés peuvent être choisis. Ce paramètre est un paramètre d'entrée pour le dispositif selon la présente divulgation. Bien sûr, la spline est déterminée sur la surface présentant les points de référence. La spline passe par un nombre fini de points se trouvant sur la surface de l'os. Elle passe par les points d'ancrage et des points définis sur la surface de l'os afin qu'elle suive la forme de l'os, par exemple des points tous les 0,5 mm.

La spline est une courbe de type géodésique passant par les points intermédiaires sur la surface de l'os. La courbe virtuelle génératrice est déterminée par l'unité de calcul, à partir des emplacements d'entrée de la pluralité de vis virtuelles et des extrémités longitudinales.

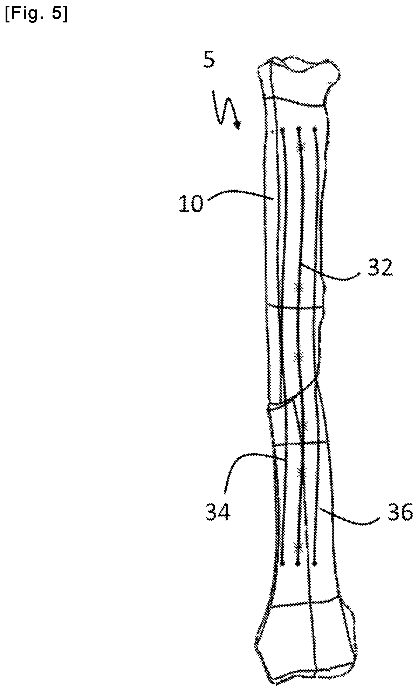

**Figure 5** représente une vue en perspective d'une représentation d'un modèle d'os avec courbes virtuelles 5. Le modèle d'os avec courbe virtuelle génératrice 5 comporte d'une part le modèle d'os 10, la courbe virtuelle génératrice 32 et deux courbes latérales 34, respectivement 36. Selon une possibilité, les courbes latérales 34, respectivement 36, sont espacées d'une distance prédéterminée de part et d'autre de la courbe virtuelle génératrice et projetées sur la surface virtuelle de l'os.. Typiquement, une distance peut être un multiple du diamètre des vis, par exemple 3 diamètres de vis.

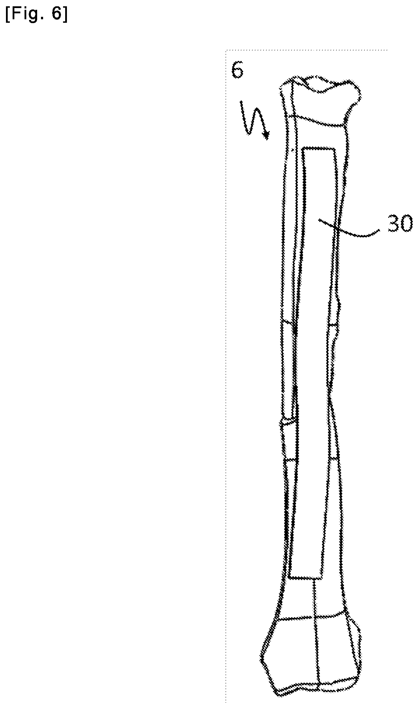

**Figure** 6 représente une vue en perspective d'une représentation d'un modèle d'os avec surface virtuelle. Le modèle d'os avec courbe virtuelle génératrice 6 comporte d'une part le modèle d'os 10, et une surface virtuelle 30. La surface virtuelle est déterminée par l'unité de calcul à partir des trois courbes virtuelles 32, 34 et 36.

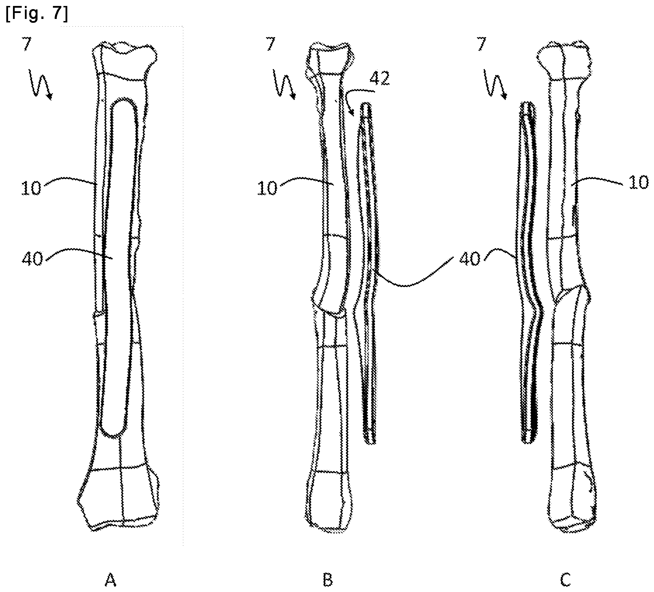

**Figure** 7 illustre un modèle d'os avec plaque d'ostéosynthèse 7. Le modèle d'os avec surface d'ostéosynthèse 7 comporte d'une part le modèle d'os 10 et d'autre part un modèle 40 de plaque d'ostéosynthèse. Les modèles d'os 10 et le modèle 40 de plaque d'ostéosynthèse sont vus de face sur la sous-figure A, de gauche sur la sous-figure B, et de droite sur la sous-figure C. Le modèle 40 de plaque d'ostéosynthèse présente une face virtuelle 42 définie par la surface virtuelle 30. Lorsque le modèle 40 est positionné sur le modèle d'os, la face virtuelle 42 et la surface virtuelle 30 coïncident.

Le modèle 40 est obtenu par mise en volume de la surface virtuelle 42. Par exemple, le modèle 40 est obtenu par extrusion de la face virtuelle 42. Par extrusion, d'une surface, on vise le procédé permettant d'obtenir un volume par translation de la surface selon un axe prédéterminé. Par exemple un axe passant par le centre du canal médullaire de l'os. Selon une autre possibilité, le modèle 40 est obtenu par remplissage entre la surface virtuelle 42 et la surface parallèle à 42 distante d'une distance prédéterminée qui est l'épaisseur du modèle 40. En outre, l'unité de calcul peut déterminer des congés au niveau des extrémités longitudinales de la surface virtuelle 30.

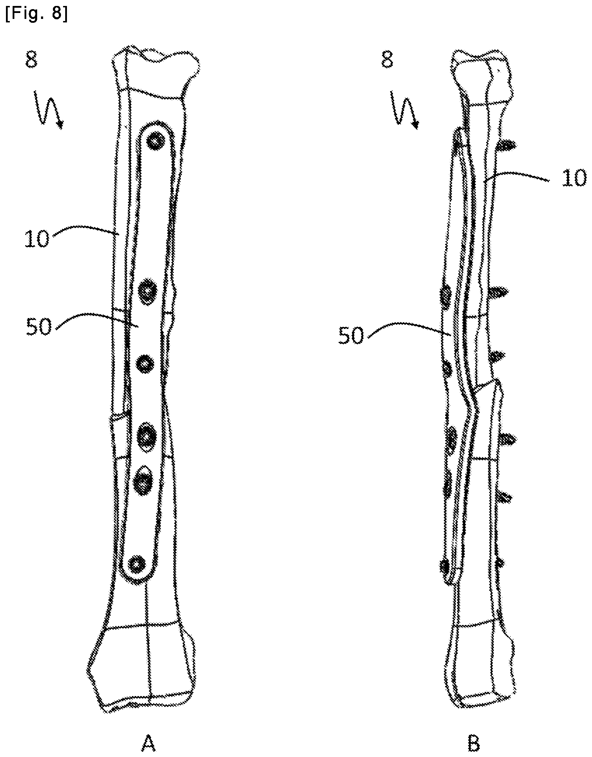

**Figure** 8 illustre un modèle d'os avec plaque d'ostéosynthèse percée et vis positionnées 8. Le modèle d'os avec surface virtuelle et vis positionnées 8 comporte d'une part le modèle d'os 10 et d'autre part un modèle 42 de plaque d'ostéosynthèse percée. Il comporte en outre les vis positionnées dans le modèle 50 et dans modèle d'os 10. Le modèle d'os avec surface virtuelle et vis positionnées 8 est vu de face sur la sous-figure A, de trois quarts droite sur la sous-figure B.

Pour générer le modèle de plaque d'ostéosynthèse percée, l'unité de calcul est configurée pour générer une représentation comportant le modèle d'os, le modèle de plaque d'ostéosynthèse virtuelle 40 plaqué sur l'os virtuel et positionné de sorte que sa face virtuelle coïncide avec la surface virtuelle 30.

Le modèle 42 de plaque d'ostéosynthèse percée est obtenu par modification du modèle 40 de plaque d'ostéosynthèse par perçage virtuel de la plaque d'ostéosynthèse sur son épaisseur pour former des trous de vis virtuels agencés pour recevoir la pluralité de vis virtuelles. Le modèle de plaque d'ostéosynthèse percée 40 peut être du type modèle de fichier en volume, par exemple au format STL ou OBJ.

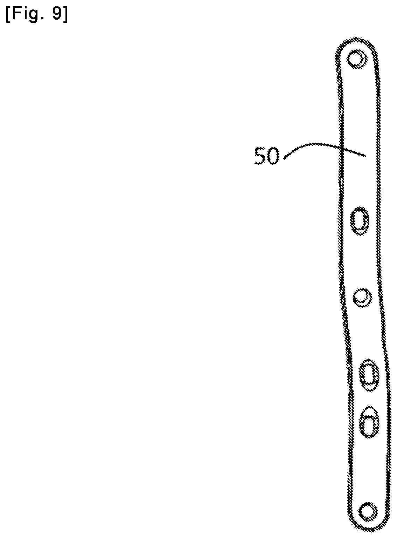

**Figure 9** est une vue d'une représentation du modèle de plaque d'ostéosynthèse percée 50 obtenue. Le modèle de plaque d'ostéosynthèse percée 40 peut être envoyé à destination d'un dispositif permettant de fabriquer une plaque d'ostéosynthèse à partir du modèle de plaque d'ostéosynthèse percée. La plaque d'ostéosynthèse fabriquée est dans un matériau de grade médical.

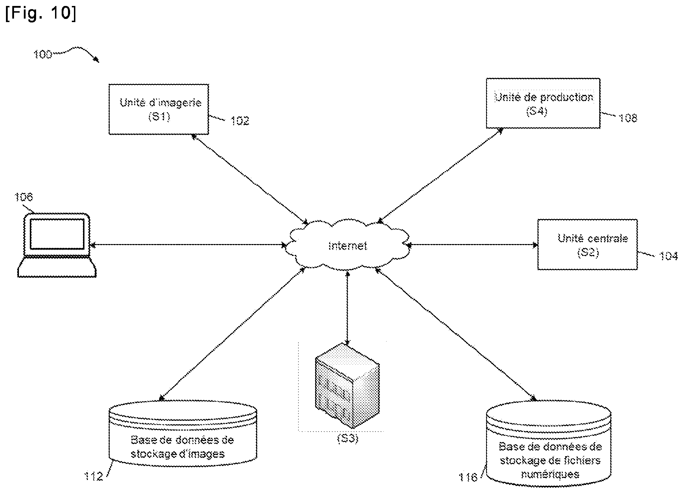

**Figure 10** est une vue schématique d'un système 100 selon la présente divulgation. Le système 100 selon la présente divulgation peut comporter un dispositif d'imagerie 102 situé dans une unité d'imagerie S1, par exemple dans un hôpital, une clinique ou une structure libérale dédiée à l'imagerie médicale. Les données tridimensionnelles de l'os réel peuvent être envoyées à une unité centrale 104 dans un lieu S2 distant du lieu S1, par exemple dans une infrastructure de réseau de type nuagique.

L'utilisateur, typiquement un chirurgien, peut se situer en un troisième lieu S3, par exemple dans son bureau et afficher sur un écran d'ordinateur 106 une représentation d'un modèle de l'os généré par l'unité centrale 104. Le dispositif 108 permettant de fabriquer la plaque d'ostéosynthèse peut être, par exemple, une imprimante 3D et être situé dans une usine de production S4, dans un lieu distinct des deux précédents lieux. L'usine de production peut par exemple comporter des logiciels interfacés avec celui d'un hôpital ou clinique, pour prévenir le logiciel de planification de l'hôpital de la prochaine disponibilité de la plaque d'ostéosynthèse.

Dans diverses implémentations, une base de données de stockage d'images 112 peut stocker des fichiers d'imagerie médicale pour une pluralité de patients/utilisateurs. La base de données de stockage d'images 112 peut être accessible à l'appareil opérateur 106, à l'appareil d'imagerie 102 (pour le téléchargement de fichiers d'imagerie), à l'unité centrale 104, à l'usine de production 108, etc. via l'Internet. Dans diverses implémentations, la base de données de stockage d'images 112 peut être située sur un réseau d'accès local de l'un des sites, par exemple, l'usine de production 108 ou un hôpital. En outre, le système 100 comprend une base de données de stockage de fichiers numériques 116, qui peut stocker des fichiers numériques générés pour la production d'une plaque osseuse ou d'un guide chirurgical pour un patient ou un utilisateur particulier. L'usine de production 108 peut obtenir les fichiers numériques du stockage de fichiers numériques 116 via l'Internet pour produire la plaque osseuse ou le guide chirurgical.

Bien sûr, les différentes caractéristiques, formes, variantes et modes de réalisation de la présente divulgation peuvent être associées les unes avec les autres selon diverses combinaisons dans la mesure où elles ne sont pas incompatibles ou exclusives les unes des autres. En particulier, toutes les variantes et modes de réalisation décrits précédemment sont combinables entre eux.

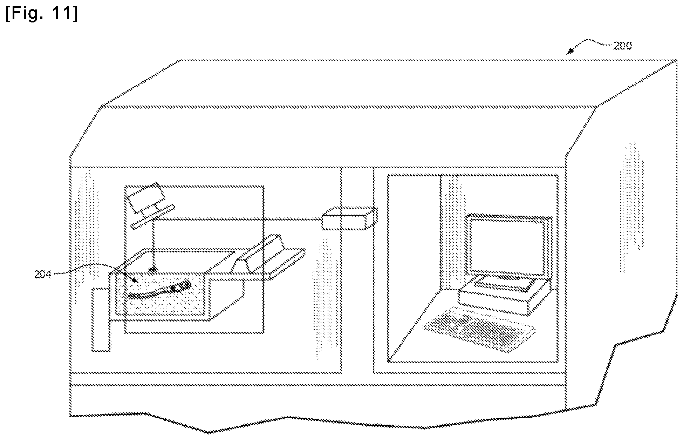

**Figure 11** est un exemple de machine de fabrication additive 200 créant une plaque d'ostéosynthèse perforée 204. Dans diverses implémentations, la machine de fabrication additive 200 reçoit un modèle de plaque d'ostéosynthèse perforée d'un dispositif séparé qui génère un modèle basé sur des images de l'os. La machine de fabrication additive 200 peut construire la plaque d'ostéosynthèse perforée 204 à l'aide du modèle de plaque d'ostéosynthèse perforée en appliquant les méthodes décrites ci-dessus.

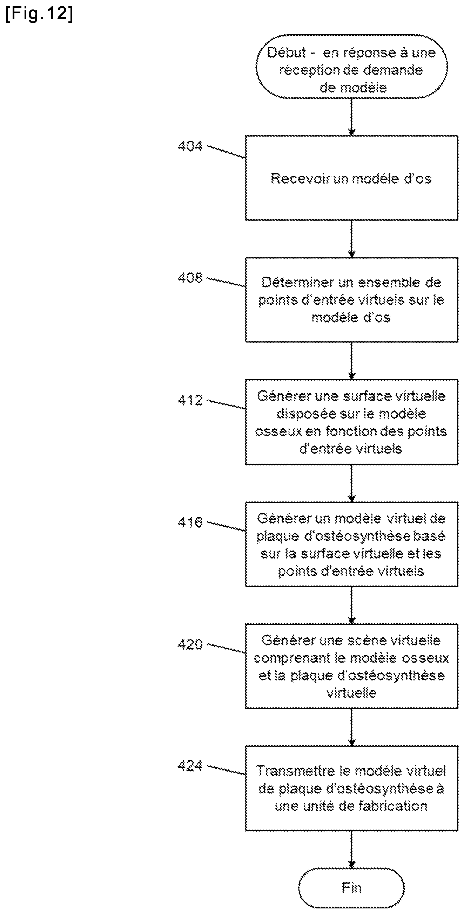

**Figure 12** est un organigramme décrivant la génération d'un modèle de plaque d'ostéosynthèse perforée. Le contrôle peut être effectué par un ensemble d'instructions stockées dans la mémoire d'un appareil et exploitées par un processeur de l'appareil. Par exemple, les instructions peuvent être exécutées par l'unité centrale 104. Le contrôle commence en réponse à la réception d'une demande de modèle, par exemple, d'un opérateur ou d'un chirurgien pour envoyer générer le modèle et envoyer le modèle pour la génération de la plaque d'ostéosynthèse perforée. À l'étape 404, le contrôle obtient un modèle osseux en 3D à partir, par exemple, d'un stockage de données qui stocke des données d'imagerie pour une pluralité d'utilisateurs. Dans diverses implémentations, la demande de modèle identifie un utilisateur ou un patient et le contrôle obtient le modèle osseux 3D correspondant à l'utilisateur. Dans diverses implémentations, le modèle osseux n'est pas en 3D, par exemple, le modèle osseux peut inclure des images 2D de l'os.

Le contrôle se poursuit jusqu'au 408 pour déterminer un ensemble d'emplacements d'entrée virtuels sur le modèle osseux. Le contrôle passe à 412 pour générer une surface virtuelle disposée sur le modèle osseux en fonction des points d'entrée virtuels. Comme décrit précédemment, la surface virtuelle peut former une forme particulière basée sur les points d'entrée virtuels. Le contrôle passe à la position 416 pour générer un modèle de plaque d'ostéosynthèse virtuelle basé sur la surface virtuelle et les points d'entrée virtuels, comme le modèle de plaque d'ostéosynthèse virtuelle illustré en Figure 9.

Le contrôle passe à 420 pour générer éventuellement une scène virtuelle comprenant le modèle osseux et la plaque d'ostéosynthèse virtuelle. Dans diverses implémentations, le contrôle saute 420 et continue à 424. À 424, le contrôle transmet le modèle de plaque d'ostéosynthèse virtuelle à un dispositif de fabrication. Dans diverses implémentations, à 424, le contrôle peut transmettre le modèle d'ostéosynthèse virtuel à un dispositif de stockage, qui peut être accessible par le dispositif de fabrication pour la production d'une plaque d'ostéosynthèse.

### Conclusions

La description qui précède est de nature purement illustrative et ne vise en aucune façon à limiter la divulgation, son application ou ses utilisations. Les enseignements généraux de la divulgation peuvent être mis en œuvre sous diverses formes. Par conséquent, bien que cette divulgation comprenne des exemples particuliers, sa portée réelle ne doit pas être aussi limitée puisque d'autres modifications apparaîtront après l'étude des dessins, de la spécification et des revendications suivantes. Il doit être entendu qu'une ou plusieurs étapes d'une méthode peuvent être exécutées dans un ordre différent (ou simultanément) sans altérer les principes de la présente divulgation. En outre, bien que chacune des incorporations soit décrite ci-dessus comme ayant certaines caractéristiques, une ou plusieurs de ces caractéristiques décrites en ce qui concerne toute incorporation de la divulgation peuvent être mises en œuvre dans et/ou combinées avec des caractéristiques de toute autre incorporation, même si cette combinaison n'est pas explicitement décrite. En d'autres termes, les formes de réalisation décrites ne s'excluent pas mutuellement et les permutations d'une ou plusieurs formes de réalisation entre elles restent dans le champ d'application de la divulgation.

Le terme « code », tel qu'il est utilisé ci-dessus, peut inclure les logiciels, les micrologiciels et/ou les microcodes, et peut se référer à des programmes, des routines, des fonctions, des classes, des structures de données et/ou des objets. Le terme circuit de processeur partagé englobe un circuit de processeur unique qui exécute tout ou partie du code de plusieurs modules. Le terme circuit de processeur groupé englobe un circuit de processeur qui, en combinaison avec des circuits de processeur supplémentaires, exécute tout ou partie du code d'un ou de plusieurs modules. Les références aux circuits à processeurs multiples englobent les circuits à processeurs multiples sur des matrices discrètes, les circuits à processeurs multiples sur une seule matrice, les noyaux multiples d'un seul circuit à processeurs, les fils multiples d'un seul circuit à processeurs, ou une combinaison de ces éléments. Le terme « circuit de mémoire partagée » englobe un circuit de mémoire unique qui stocke une partie ou la totalité du code de plusieurs modules. Le terme circuit de mémoire collective englobe un circuit de mémoire qui, en combinaison avec des mémoires supplémentaires, stocke tout ou partie du code d'un ou de plusieurs modules.

Les appareils et méthodes décrits dans la présente application peuvent être partiellement ou totalement mis en œuvre par un ordinateur spécialisé créé en configurant un ordinateur général pour exécuter une ou plusieurs fonctions particulières incorporées dans des programmes informatiques. Les blocs fonctionnels et les éléments d'organigramme décrits ci-dessus servent de spécifications logicielles, qui peuvent être traduites dans les programmes informatiques par le travail de routine d'un technicien ou d'un programmeur qualifié.

Les programmes informatiques comprennent des instructions exécutables par le processeur qui sont stockées sur au moins un support non transitoire lisible par l'ordinateur. Les programmes informatiques peuvent également inclure des données stockées ou s'appuyer sur celles-ci. Les programmes informatiques peuvent comprendre un système d'entrée/sortie de base (BIOS) qui interagit avec le matériel de l'ordinateur spécialisé, des pilotes de périphériques qui interagissent avec des périphériques particuliers de l'ordinateur spécialisé, un ou plusieurs systèmes d'exploitation, des applications utilisateur, des services d'arrière-plan, des applications d'arrière-plan, etc.

Les programmes informatiques peuvent inclure : (i) du texte descriptif à analyser, tel que HTML (hypertext markup language), XML (extensible markup language), ou JSON (JavaScript Object Notation), (ii) du code assembleur, (iii) du code objet généré à partir du code source par un compilateur, (iv) du code source pour exécution par un interprète, (v) du code source pour compilation et exécution par un compilateur juste à temps, etc. À titre d'exemple uniquement, le code source peut être écrit en utilisant la syntaxe de langages tels que C, C++, C#, Objective C, Swift, Haskell, Go, SQL, R, Lisp, Java^{®}, Fortran, Perl, Pascal, Curl, OCaml, JavaScript^{®}, HTML5 (Hypertext Markup Language 5e révision), Ada, ASP (Active Server Pages), PHP (PHP : Hypertext Preprocessor), Scala, Eiffel, Smalltalk, Erlang, Ruby, Flash^{®}, Visual Basic^{®}, Lua, MATLAB, SIMULINK, et Python^{®}.

## Revendications

1. Procédé de génération d'une plaque d'ostéosynthèse convenant à être fixée sur un os comportant une unité de calcul configurée pour mettre en œuvre les étapes suivantes :
• obtenir (404) un modèle d'os (10) représentant un os généré à partir de données d'imagerie correspondant à l'os à partir d'un dispositif d'imagerie ;
• déterminer (408) un ensemble de points d'entrée virtuels (12, 14) sur le modèle osseux ;
• générer (412) une surface virtuelle (30) adjacente au modèle osseux sur la base du modèle osseux et de l'ensemble des points d'entrée virtuels ;
• générer (416) un modèle de plaque d'ostéosynthèse (40) basé sur la surface virtuelle ;
• créer un trou virtuel à chaque point d'entrée virtuel de l'ensemble des points d'entrée virtuels le long du modèle de plaque d'ostéosynthèse ; et
• transmettre (420) le modèle de plaque d'ostéosynthèse à une machine de fabrication (108) de plaques d'ostéosynthèse.

2. Procédé selon la revendication 1, dans lequel l'ensemble des points d'entrée virtuels (12) indiquent les endroits où une vis est insérée dans l'os.

3. Procédé selon la revendication 1 ou 2, dans lequel le modèle osseux (10) est tridimensionnel.

4. Procédé selon l'une quelconque des revendications précédentes, comportant en outre une étape d'obtention de données d'imagerie correspondant à l'os à partir d'un dispositif d'imagerie (102).

5. Procédé selon l'une quelconque des revendications précédentes, dans lequel la surface virtuelle comprend l'ensemble des emplacements d'entrée virtuels, une vis est l'une des multiples vis et le trou virtuel est l'un des multiples trous.

6. Procédé selon l'une quelconque des revendications précédentes, dans lequel le modèle de plaque d'ostéosynthèse (40) comprend une face virtuelle définie par la surface virtuelle (30) et une épaisseur définie par une extrusion de la face virtuelle.

7. Procédé selon l'une quelconque des revendications précédentes, comportant en outre :
• la génération (420) d'une scène virtuelle comprenant le modèle d'os, dans laquelle le modèle de plaque d'ostéosynthèse est pressé contre le modèle d'os et positionné de telle sorte qu'une face virtuelle (42) du modèle de plaque d'ostéosynthèse (40) coïncide avec la surface virtuelle (30),
dans lequel la génération d'un modèle de plaque d'ostéosynthèse et la fabrication ultérieure d'une plaque d'ostéosynthèse sur mesure évite au chirurgien de devoir modifier la plaque d'ostéosynthèse pendant l'opération.

8. Procédé selon l'une quelconque des revendications précédentes, dans lequel la surface virtuelle est déterminée électroniquement à partir d'une courbe génératrice virtuelle (32) qui passe successivement par des points de référence, les points de référence étant déterminés comme les centres de l'ensemble des points d'entrée virtuels et d'au moins deux courbes virtuelles latérales qui sont disposées de chaque côté de la courbe génératrice virtuelle.

9. Procédé selon la revendication précédente, dans lequel les points de référence comprennent des extrémités longitudinales (14), la courbe génératrice virtuelle (32) passant successivement par l'une des extrémités longitudinales, les centres de l'ensemble des points d'entrée virtuels, et une autre des extrémités longitudinales.

10. Procédé selon la revendication 8, dans laquelle la courbe génératrice virtuelle (32) est une spline de type géodésique passant par les points de référence.

11. Procédé selon la revendication 8, dans lequel les courbes virtuelles latérales (34, 36) sont des splines de type géodésique qui passent par des images des points de référence, et dans lequel les images sont obtenues par translation d'une distance qui est fonction d'une largeur d'une vis à un diamètre maximal perpendiculaire à un axe du modèle de plaque d'ostéosynthèse et tangente à la surface virtuelle du modèle d'os.

12. Procédé selon la revendication 8, dans lequel les courbes virtuelles latérales (34, 36) sont des courbes virtuelles espacées d'une distance prédéterminée de part et d'autre de la courbe virtuelle génératrice (32) et projetées sur la surface virtuelle de l'os.

13. Procédé selon l'une quelconque des revendications précédentes, comportant en outre :
• une détermination d'un modèle de plaque d'ostéosynthèse intermédiaire en ajoutant des congés virtuels au modèle de plaque d'ostéosynthèse aux extrémités longitudinales du modèle de plaque d'ostéosynthèse, dans lequel la détermination du modèle de plaque d'ostéosynthèse est effectuée en modifiant le modèle de plaque d'ostéosynthèse intermédiaire.

14. Procédé selon l'une quelconque des revendications précédentes, dans lequel la machine de fabrication de plaques d'ostéosynthèse est une machine de fabrication additive ou soustractive.

15. Procédé selon l'une quelconque des revendications précédentes, dans lequel la plaque d'ostéosynthèse qui est fabriquée est faite de matériaux de grade médical.

16. Procédé selon l'une quelconque des revendications précédentes, dans lequel la machine de fabrication de plaques d'ostéosynthèse utilise le frittage laser ou l'impression tridimensionnelle et se trouve à un endroit éloigné de la modélisation.

17. Produit programme d'ordinateur comprenant des instructions qui, lorsque le programme est exécuté par un ordinateur, conduisent celui-ci à mettre en œuvre les étapes du procédé selon l'une quelconque des revendications précédentes.

18. Support d'enregistrement lisible par ordinateur comprenant des instructions qui, lorsqu'elles sont exécutées par un ordinateur, conduisent celui-ci à mettre en œuvre les étapes du procédé selon l'une quelconque des revendications précédentes de procédé.

## Patentansprüche

1. Verfahren zur Herstellung einer Osteosyntheseplatte, die zur Befestigung an einem Knochen geeignet ist, umfassend eine Recheneinheit, die so eingerichtet ist, dass sie die folgenden Schritte,ausführt:
- Erhalten (404) eines Knochenmodells (10), das einen Knochen darstellt, erzeugt aus Bildgebungsdaten, die dem Knochen von einem Bildgebungsgerät entsprechen;
- Bestimmen (408) eines Satzes virtueller Eintrittspunkte (12, 14) auf dem Knochenmodell;
- Erzeugen (412) einer virtuellen Fläche (30) angrenzend an das Knochenmodell basierend auf dem Knochenmodell und dem Satz virtueller Eintrittspunkte;
- Erzeugen (416) eines Osteosyntheseplattenmodells (40) basierend auf der virtuellen Fläche;
- Erstellen eines virtuellen Lochs an jedem virtuellen Eintrittspunkt des Satzes virtueller Eintrittspunkte entlang des Osteosyntheseplattenmodells; und
- Übermitteln (420) des Osteosyntheseplattenmodells an eine Plattenherstellungsmaschine (108).

2. Verfahren nach Anspruch 1, wobei der Satz virtueller Eintrittspunkte (12) die Stellen angibt, an denen eine Schraube in den Knochen eingesetzt wird.

3. Verfahren nach Anspruch 1 oder 2, wobei das Knochenmodell (10) dreidimensional ist.

4. Verfahren nach einem der vorstehenden Ansprüche, ferner umfassend einen Schritt des Erhaltens von Bildgebungsdaten, die dem Knochen von einem Bildgebungsgerät (102) entsprechen.

5. Verfahren nach einem der vorstehenden Ansprüche, wobei die virtuelle Fläche alle virtuellen Eintrittsstellen umfasst, eine Schraube eine von mehreren Schrauben ist, und das virtuelle Loch eines von mehreren Löchern ist.

6. Verfahren nach einem der vorstehenden Ansprüche, wobei das Osteosyntheseplattenmodell (40) eine virtuelle Fläche aufweist, die durch die virtuelle Fläche (30) definiert ist, und eine Dicke, die durch eine Extrusion der virtuellen Fläche definiert ist.

7. Verfahren nach einem der vorstehenden Ansprüche, ferner umfassend:
- Erzeugen (420) einer virtuellen Szene, die das Knochenmodell umfasst, wobei das Osteosyntheseplattenmodell gegen das Knochenmodell gedrückt und so positioniert wird, dass eine virtuelle Fläche (42) des Osteosyntheseplattenmodells (40) mit der virtuellen Fläche (30) übereinstimmt, wobei das Erzeugen eines Osteosyntheseplattenmodells und anschließend die Herstellung einer maßgeschneiderten Osteosyntheseplatte verhindert, dass der Chirurg die Osteosyntheseplatte während der Operation modifizieren muss.

8. Verfahren nach einem der vorstehenden Ansprüche, wobei die virtuelle Fläche elektronisch aus einer virtuellen Erzeugungskurve (32) bestimmt wird, die nacheinander durch Referenzpunkte verläuft, wobei die Referenzpunkte als Zentren des Satzes virtueller Eintrittspunkte und mindestens zwei seitliche virtuelle Kurven angeordnet auf jeder Seite der virtuellen Erzeugungskurve bestimmt werden.

9. Verfahren nach Anspruch 8, wobei die Referenzpunkte Längsenden (14) umfassen, wobei die virtuelle Erzeugungskurve (32) nacheinander durch eines der Längsenden, die Zentren des Satzes virtueller Eintrittspunkte und ein anderes der Längsenden verläuft.

10. Verfahren nach Anspruch 8, wobei die virtuelle Erzeugungskurve (32) eine geodätische Art von Spline ist, die durch die Referenzpunkte verläuft.

11. Verfahren nach den Ansprüchen, wobei die seitlichen virtuellen Kurven (34, 36) geodätische Splines sind, die durch Bilder der Referenzpunkte verlaufen, und wobei die Bilder durch Translation über eine Distanz erhalten werden, die eine Funktion des Durchmessers einer Schraube im maximalen Durchmesser ist, senkrecht zu einer Achse des Osteosyntheseplattenmodells und tangential zur virtuellen Fläche des Knochenmodells.

12. Verfahren nach Anspruch 8, wobei die seitlichen virtuellen Kurven (34, 36) virtuelle Kurven sind, die in einem vorgegebenen Abstand auf beiden Seiten der virtuellen Erzeugungskurve (32) angeordnet und auf die virtuelle Fläche des Knochens projiziert sind.

13. Verfahren nach einem der vorstehenden Ansprüche, ferner umfassend:
- Bestimmen eines Zwischen-Osteosyntheseplattenmodells durch Hinzufügen virtueller Radien zu dem Osteosyntheseplattenmodell an den Längsenden des Osteosyntheseplattenmodells, wobei die Bestimmung des Osteosyntheseplattenmodells durch Modifizieren des Zwischen-Osteosyntheseplattenmodells erfolgt.

14. Verfahren nach einem der vorstehenden Ansprüche, wobei die Osteosyntheseplattenherstellungsmaschine eine additive oder subtraktive Herstellungsmaschine ist.

15. Verfahren nach einem der vorstehenden Ansprüche, wobei die hergestellte Osteosyntheseplatte aus medizinisch geeigneten Materialien besteht.

16. Verfahren nach einem der vorstehenden Ansprüche, wobei die Osteosyntheseplattenherstellungsmaschine Lasersintern oder Dreidruck verwendet und an einem Standort entfernt von der Modellierung angeordnet ist.

17. Computerprogrammprodukt, das Anweisungen umfasst, die, wenn sie von einem Computer ausgeführt werden, den Computer veranlassen, die Schritte des Verfahrens nach einem der vorstehenden Ansprüche auszuführen.

18. Computerlesbares Speichermedium, das Anweisungen umfasst, die, wenn sie von einem Computer ausgeführt werden, den Computer veranlassen, die Schritte des Verfahrens nach einem der vorstehenden Ansprüche auszuführen.

## Claims

1. Method for generating an osteosynthesis plate suitable for being fixed to a bone, comprising a computing unit configured to implement the following steps:
• obtaining (404) a bone model (10) representing a bone generated from imaging data corresponding to the bone from an imaging device;
• determining (408) a set of virtual entry points (12, 14) on the bone model;
• generating (412) a virtual surface (30) adjacent to the bone model based on the bone model and the set of virtual entry points;
• generating (416) an osteosynthesis plate model (40) based on the virtual surface;
• creating a virtual hole at each virtual entry point of the set of virtual entry points along the osteosynthesis plate model; and
• transmitting (420) the osteosynthesis plate model to a plate manufacturing machine (108).

2. Method according to claim 1, wherein the set of virtual entry points (12) indicates the locations where a screw is inserted into the bone.

3. Method according to claim 1 or 2, wherein the bone model (10) is three-dimensional.

4. Method according to any one of the preceding claims, further comprising a step of obtaining imaging data corresponding to the bone from an imaging device (102).

5. Method according to any one of the preceding claims, wherein the virtual surface includes all the virtual entry locations, a screw is one of multiple screws, and the virtual hole is one of multiple holes.

6. Method according to any one of the preceding claims, wherein the osteosynthesis plate model (40) comprises a virtual face defined by the virtual surface (30) and a thickness defined by an extrusion of the virtual face.

7. Method according to any one of the preceding claims, further comprising:
• generating (420) a virtual scene comprising the bone model, wherein the osteosynthesis plate model is pressed against the bone model and positioned so that a virtual face (42) of the osteosynthesis plate model (40) coincides with the virtual surface (30),
wherein generating an osteosynthesis plate model and subsequently manufacturing a customized osteosynthesis plate avoids the surgeon having to modify the osteosynthesis plate during surgery.

8. Method according to any one of the preceding claims, wherein the virtual surface is electronically determined from a virtual generating curve (32) that passes successively through reference points, the reference points being determined as the centers of the set of virtual entry points and at least two lateral virtual curves arranged on each side of the virtual generating curve.

9. Method according to the preceding claim, wherein the reference points include longitudinal ends (14), the virtual generating curve (32) passing successively through one of the longitudinal ends, the centers of the set of virtual entry points, and another of the longitudinal ends.

10. Method according to claim 8, wherein the virtual generating curve (32) is a geodesic-type spline passing through the reference points.

11. Method according to the claims, wherein the lateral virtual curves (34, 36) are geodesic-type splines passing through images of the reference points, and wherein the images are obtained by translation over a distance that is a function of the width of a screw at a maximum diameter, perpendicular to an axis of the osteosynthesis plate model and tangent to the virtual surface of the bone model.

12. Method according to claim 8, wherein the lateral virtual curves (34, 36) are virtual curves spaced at a predetermined distance on either side of the virtual generating curve (32) and projected onto the virtual surface of the bone.

13. Method according to any one of the preceding claims, further comprising:
• determining an intermediate osteosynthesis plate model by adding virtual fillets to the osteosynthesis plate model at the longitudinal ends of the osteosynthesis plate model, wherein the determination of the osteosynthesis plate model is carried out by modifying the intermediate osteosynthesis plate model.

14. Method according to any one of the preceding claims, wherein the osteosynthesis plate manufacturing machine is an additive or subtractive manufacturing machine.

15. Method according to any one of the preceding claims, wherein the osteosynthesis plate being manufactured is made of medical-grade materials.

16. Method according to any one of the preceding claims, wherein the osteosynthesis plate manufacturing machine uses laser sintering or three-dimensional printing and is located at a site remote from the modeling.

17. Computer program product comprising instructions which, when executed by a computer, cause the computer to implement the steps of the method according to any one of the preceding claims.

18. Computer-readable storage medium comprising instructions which, when executed by a computer, cause the computer to implement the steps of the method according to any one of the preceding claims.
